# EUROPÄISCHE PATENTSCHRIFT

(11) **EP 2 177 153 B1**
(45) Veröffentlichungstag und Bekanntmachung des Hinweises auf die Patenterteilung: **10.10.2012**
(21) Anmeldenummer: 09173182.8
(22) Anmeldetag: 15.10.2009
(51) Int. Cl.: A61B 5/00

(54) **Erkennen einer Lungenkrebserkrankung mithilfe eines Hundes**
Detection of lung cancer using a dog
Reconnaissance d'un cancer des poumons à l'aide d'un chien

(30) Priorität: 16.10.2008 AT 16242008
(43) Veröffentlichungstag der Anmeldung: 21.04.2010
(73) Patentinhaber: Darwin GmbH, 8740 Zeltweg (AT)
(72) Erfinder: Gleichweit, Wolfgang, 8130, Frohnleiten (AT)
(74) Vertreter: Wirnsberger, Gernot

(56) Entgegenhaltungen:
- US-A1- 2005 065 446
- MCCULLOCH MICHAEL ET AL: "Diagnostic accuracy of canine scent detection in early- and late-stage lung and breast cancers." INTEGRATIVE CANCER THERAPIES MAR 2006, Bd. 5, Nr. 1, März 2006 (2006-03), Seiten 30-39, XP002560253 ISSN: 1534-7354
- MACHADO ROBERTO F ET AL: "Detection of lung cancer by sensor array analyses of exhaled breath.", AMERICAN JOURNAL OF RESPIRATORY AND CRITICAL CARE MEDICINE 1 JUN 2005 LNKD- PUBMED:15750044, vol. 171, no. 11, 1 June 2005 (2005-06-01) , pages 1286-1291, ISSN: 1073-449X
- AMORIM L C A ET AL: "Breath air analysis and its use as a biomarker in biological monitoring of occupational and environmental exposure to chemical agents", JOURNAL OF CHROMATOGRAPHY B: ANALYTICAL TECHNOLOGIES IN THE BIOMEDICAL AND LIFE SCIENCES 20070615 ELSEVIER NL, vol. 853, no. 1-2, 15 June 2007 (2007-06-15), pages 1-9, DOI: DOI:10.1016/J.JCHROMB.2007.03.023
- STATHEROPOULOS M ET AL: "Preliminary investigation of using volatile organic compounds from human expired air, blood and urine for locating entrapped people in earthquakes", JOURNAL OF CHROMATOGRAPHY B: BIOMEDICAL SCIENCES & APPLICATIONS, ELSEVIER, AMSTERDAM, NL, vol. 822, no. 1-2, 5 August 2005 (2005-08-05), pages 112-117, XP027627153, ISSN: 1570-0232 [retrieved on 2005-08-05]

## Beschreibung

Die Erfindung betrifft ein Verfahren zur Gewinnung einer Probe zum Erkennen einer Lungenkrebserkrankung mithilfe eines Hundes.

Lungenkrebserkrankungen, die beispielsweise auf regelmäßigen Konsum von Zigaretten zurückzuführen sind, sind heute eine häufige Todesursache von Menschen. Eine Lungenkrebserkrankung kann zwar unter Einsatz geeigneter Hilfsmittel bzw. Apparaturen bereits in einem frühen Stadium diagnostiziert werden, allerdings kommt in der Regel aus Kostengründen vor einer eingehenden Untersuchung eine bloße Durchleuchtung einer Lunge eines Menschen bzw. Patienten mit Röntgenstrahlen zum Einsatz. Dabei ist allerdings nachteilig, dass mit diesem Verfahren lediglich bereits relativ große Geschwüre, die auf eine Lungenkrebserkrankung zurückzuführen sind, detektiert werden können. Kleinere Geschwüre in einem früheren Stadium sind zwar wie erwähnt mit aufwendigeren Verfahren bzw. Apparaturen nachweisbar, allerdings werden solche Verfahren bzw. Apparaturen aufgrund hoher Kosten bei einer Erstuntersuchung in der Regel nicht eingesetzt.

Hier setzt die Erfindung an. Aufgabe der Erfindung ist es, ein Verfahren anzugeben, auf möglichst einfache Weise eine Probe zum Erkennen einer Lungenkrebserkrankung mittels eines Hundes bereitzustellen, welches Ziel durch ein Verfahren gemäß Anspruch 1 erreicht wird.

Die mit der Erfindung erzielten Vorteile sind insbesondere darin zu sehen, dass mithilfe eines Hundes, der naturgemäß einen besonders guten Geruchssinn hat, auf einfache Weise mit einer gewissen Wahrscheinlichkeit festgestellt werden kann, ob es zweckmäßig ist, einen Patienten einer aufwendigeren Untersuchung zuzuführen, sodass aufwendige Untersuchungs- und Diagnostizierverfahren kosteneffizient angewendet werden können. Als Normatemluft ist eine Atemluft eines Menschen zu verstehen, bei dem eine Lungenkrebserkrankung auch mit aufwendigsten Verfahren nicht nachweisbar ist.

Bevorzugt ist es, dass mehrere weitere Behältnisse, die ein Adsorptionsmittel enthalten, das jeweils mit Atemluft eines ersten Menschen in Kontakt stand, in einer geraden Linie angeordnet werden. In diesem Fall ist es möglich, dass der eingesetzte Hund sogleich mehrere Behältnisse überprüft. Dabei ist es insbesondere zweckmäßig, dass mehrere erste Behältnisse vor den weiteren Behältnissen angeordnet werden, damit der Hund die aufzuspürenden Gerüche erkennt.

Versuche haben gezeigt, dass in mehr als 70 % eine nachfolgende nähere Untersuchung sowie Diagnose mit einer Bewertung durch den Hund übereinstimmt, sodass, auch wenn das Ergebnis in Einzelfällen nicht zutreffend sein mag, insgesamt doch mit hoher Wahrscheinlichkeit bei Anschlagen des Hundes eine nachfolgende genauere Untersuchung und Diagnose zu einem positiven Befund führt. Dies wiederum resultiert darin, dass gegebenenfalls eine Behandlung einer möglichen Lungenkrebserkrankung rechtzeitig in Angriff genommen werden kann, sodass die Behandlung auch zu einem erfolgreichen Abschluss einer Therapie im Sinne einer Heilung führt.

Zweckmäßig kann es sein, dass mehrere weitere Behältnisse, die ein Adsorptionsmittel enthalten, das jeweils mit Atemluft eines ersten Menschen in Kontakt stand, in einer geraden Linie angeordnet werden. Es ist dann möglich, durch den Hund mehrere Behältnisse bzw. Proben bewerten zu lassen. Im Zusammenhang damit ist es besonders bevorzugt, dass mehrere erste Behältnisse vor den weiteren Behältnissen angeordnet werden, sodass der Hund die festzustellenden Gerüche leicht erkennt.

Zweckmäßig ist es, damit der Hund besonders rasch anschlägt, dass dieser vor Beschnuppern einzelner Proben darauf konditioniert wird, eine mögliche Lungenkrebserkrankung eines Menschen zu erkennen.

Die mit einem solchen Verfahren erzielten Vorteile sind insbesondere darin zu sehen, dass ein Hund mit einfachen Mitteln so trainiert wird, dass dieser auf einfache Weise mit einer gewissen Wahrscheinlichkeit durch sein Verhalten eine richtige Aussage bezüglich einer möglichen Lungenkrebserkrankung eines Menschen anzeigen kann, und zwar anhand einer mit geringem Aufwand herzustellenden Probe einer Atemluft, die mit einem Adsorptionsmittel in Kontakt gebracht wurde.

Von Vorteil ist es, dass die Behältnisse in einer geraden Linie angeordnet werden, damit der Hund einzelne Behältnisse nicht voneinander unterscheiden kann.

Vorgesehen kann auch sein, dass die ersten und/oder weiteren Behältnisse als solche für den Hund nicht erkennbar gekennzeichnet werden, um allfällige Fehler beim Konditionieren des Hundes möglichst auszuschließen.

Die mit einem erfindungsgemäßen Verfahren zur Gewinnung einer Probe zum Erkennen einer Lungenkrebserkrankung mittels eines Hundes erreichten Vorteile sind insbesondere darin zu sehen, dass auf einfache und kostengünstige Weise mit hoher Wahrscheinlichkeit eine ein richtiges Ergebnis bringende Probe gewonnen werden kann, aufgrund welcher, nach Bewertung durch einen Hund, ein potenziell an Lungenkrebs erkrankter Mensch gegebenenfalls einer näheren Untersuchung und Diagnose zugeführt werden kann. Ein Verfahren zur Erkennung von Lungenkrebs mittels eines Hundes ist bekannt aus Mc Culloch, Integrative Cancer Therapies 5 (1); 2006, Seiten 30-39. Weiterer Stand der Technik ist.
Machado, Am. J. Crit. Care Med. Vol. 171; 2005, Seiten 1286-1291.

Im Folgenden ist die Erfindung anhand eines Ausführungsbeispiels noch näher erläutert.

Von an Lungenkrebs erkrankten lebenden Menschen wurde jeweils eine Atemluft in einem Plastiksack gesammelt, wobei ein Volumen des Plastiksacks jeweils vier Liter betrug. Grundsätzlich können Säcke mit einem Volumen von zwei bis zehn Litern eingesetzt werden. Die in den Plastiksäcken gesammelte Atemluft wurde anschließend jeweils durch ein beidseitig offenes Röhrchen gepresst, welches mit Aktivkohle und Silicagel gefüllt war. Die Röhrchen wurden beidseitig verschlossen und bei einer Temperatur von etwa 4 °C oder weniger gelagert.

In einem separaten Schritt wurden gleich aussehende Röhrchen bereitgestellt, die ebenfalls mit Aktivkohle und Silicagel gefüllt wurden, jedoch mit Atemluft von gemäß Diagnose nach eingehender Untersuchung nicht an Lungenkrebs erkrankten Menschen beaufschlagt wurden.

Die mit Atemluft von an Lungenkrebs erkrankten Menschen beaufschlagten Röhrchen wie auch die mit Atemluft von nicht an Lungenkrebs erkrankten Menschen beaufschlagten Röhrchen bzw. Blindproben wurden anschließend verwendet, um einen Hund so lange zu trainieren bzw. konditionieren, bis dieser bei den Röhrchen, die mit Atemluft von an Lungenkrebs erkrankten Menschen beaufschlagt waren, anschlug bzw. bellte.

Nachdem der Hund konditioniert war, wurden von verschiedenen Testpersonen Atemluftproben in der zuvor beschriebenen Weise entnommen und entsprechende Röhrchen in einer Linie angeordnet. Der konditionierte Hund wurde jeweils zu einem Röhrchen geführt, welches dann geöffnet wurde, sodass der Hund daran schnuppern konnte. Jene Röhrchen, bei welchen der Hund anschlug bzw. bellte, wurden gekennzeichnet. Jene Testpersonen, bei deren Röhrchen der Hund anschlug bzw. bellte, wurden anschließend genau auf Lungenkrebs untersucht, wobei sich in mehr als 70 % der Fälle zeigte, dass Lungenkrebs im Frühstadium diagnostiziert werden konnte.

Ähnliche Versuchsergebnisse wurden auch erhalten, wenn der Hund nicht konditioniert war, aber vor den zu testenden Röhrchen etwa drei Röhrchen angeordnet waren, welche mit Atemluft eines an Lungenkrebs erkrankten Menschen beaufschlagt waren und dem Hund vorerst zum Beschnuppern präsentiert wurden. Der Hund schlug dann ebenfalls bei Röhrchen an, die mit Atemluft von mit überwiegender Wahrscheinlichkeit an Lungenkrebs erkrankten Menschen beaufschlagt waren. Es wird vermutet, dass die mit einer Lungenkrebserkrankung verbundene Entartung menschlichen Gewebes zu einer geringfügigen Änderung der Atemluft führt, was vom Hund aufgrund dessen Geruchssinns erkannt werden kann.

## Patentansprüche

1. Verfahren zur Gewinnung einer Probe zum Erkennen einer Lungenkrebserkrankung mittels eines Hundes, umfassend folgende Schritte:
a) Sammeln einer Atemluft eines Menschen in einem aufblasbaren Sack, der ein Volumen von 2 bis 10 Liter aufweist;
b) In-Kontakt-Bringen der gesammelten Atemluft mit einem in einem Röhrchen gelagerten Adsorptionsmittel enthaltend Aktivkohle und oder Silicagel, indem die gesammelte Atemluft durch das Röhrchen gepresst wird;
c) beidseitiges Verschließen und Aufbewahren des Adsorptionsmittels im Röhrchen bei einer Temperatur von weniger als 4 °C.

2. Verfahren nach Anspruch 1, wobei das Adsorptionsmittel aus Aktivkohle und Silicagel besteht.

## Claims

1. A method for obtaining a sample for the detection of lung cancer by means of a dog, comprising the following steps:
a) collecting respiratory air of a human in an inflatable bag, which has a volume of 2 to 10 litres;
b) bringing the collected respiratory air in contact with an absorption agent containing active carbon and/or silica gel, by the collected respiratory air being pressed through the tube;
c) sealing on both sides and storage of the adsorption agent in the tube at a temperature of less than 4 °C.

2. The method according to Claim 1, wherein the adsorption agent consists of active carbon and silica gel.

## Revendications

1. Procédé permettant d'obtenir un échantillon pour détecter un cancer du poumon au moyen d'un chien, comprenant les étapes suivantes consistant à :
a) recueillir l'air inhalé par une personne dans un sac gonflable d'un volume de 2 à 10 litres ;
b) mettre en contact l'air inhalé recueilli avec un adsorbant stocké dans un tube et comprenant du charbon actif et/ou du gel de silice, en ce que l'air inhalé recueilli est comprimé à travers le tube ;
c) fermer des deux côtés et conserver l'adsorbant dans le tube à une température de moins de 4 °C.

2. Procédé selon la revendication 1, dans lequel l'adsorbant se compose de charbon actif et de gel de silice.
